# EUROPEAN PATENT APPLICATION

(11) **EP 4 275 692 A2**
(43) Date of publication of application: **15.11.2023**
(21) Application number: 22736914.7
(22) Date of filing: 10.01.2022
(51) Int. Cl.: A61K 35/32, A61P 19/02

(54) **DRIED-CELL-SECRETOME-BASED THERAPEUTIC AGENT FOR TREATING OSTEOARTHRITIS**

(30) Priority: 08.01.2021 KR 20210002725
(71) Applicant: Seoul National University R & DB Foundation, Seoul 08826 (KR)
(72) Inventor: HWANG, Nathaniel Suk-Yeon, Seoul 08826 (KR); LEE, Youn-Woo, Seoul 08826 (KR); KOH, Heeseung, Seoul 08826 (KR); PARK, Hee Jeong, Seoul 06714 (KR); KIM, Jeong Uk, Ulsan 44680 (KR)
(74) Representative: Ström & Gulliksson AB
(86) International application number: PCT/KR2022/000401
(87) International publication number: WO 2022/149937

(57) **Abstract**

The present invention relates to a method for producing a culture medium-derived component or a crystallized culture medium using a PCA (Precipitation with Compressed Fluid Anti-solvent) drying process, a method for restoring chondrocytes using the culture medium-derived component or the crystallized culture medium prepared by way of the above method, a method for treating a subject having osteoarthritis by way of the method for restoring chondrocytes using the culture medium-derived component or the crystallized culture medium, a method for treating a subject having osteoarthritis using the culture medium-derived component or the crystallized culture medium, and the use of the culture medium-derived component or the crystallized culture medium for the treatment of osteoarthritis. The culture medium-derived component provided in the present invention has excellent activity of alleviating inflammatory environment and inhibiting expression of catabolism-related genes, and the culture medium-derived component can be produced in a higher yield by way of the method provided in the present invention, and thus can be widely used in the development of therapeutic agents for osteoarthritis.

## Description

### [Technical Field]

The present invention relates to a dried cell secretome-based therapeutic agent for osteoarthritis treatment, and more specifically to a method for producing a culture medium-derived component or a crystallized culture medium using a PCA (Precipitation with Compressed Fluid Anti-solvent) drying process, a method for restoring chondrocytes using the culture medium-derived component or the crystallized culture medium prepared by way of the above method, a method for treating a subject having osteoarthritis by way of the method for restoring chondrocytes using the culture medium-derived component or the crystallized culture medium, and a method for treating a subject having osteoarthritis using the culture medium-derived component or the crystallized culture medium.

### [Background Art]

The human body maintains a balance between oxidative and reductive reactions. When the body loses this balance and is inclined to promote oxidation due to various factors, oxidative stress is induced in the body, damaging cells and causing pathological diseases. Reactive oxygen species (ROS), which are a direct cause of oxidative stress, are chemically unstable and highly reactive, and thus they can easily react with various biomaterials such as DNA, proteins, lipids, and carbohydrates, and attack polymers in the body, thereby leading to irreversible damage to cells and tissues or causing mutation, cytotoxicity, cancer, *etc.*

Meanwhile, macrophages in the human body produce inflammatory cytokines, such as tumor necrotic factor-α (TNF-α), interleukin-6 (IL-6), interleukin-1β (IL-1β), *etc.,* in response to pathogens, and produce nitric oxide (NO) and prostaglandins (prostaglandin E2, PGE2) by synthesizing inducible nitric oxide synthase (iNOS) and cyclooxygenase-2 (COX-2). At physiological level, nitric oxide (NO) has various roles in removing bacteria and tumors, regulating blood pressure, mediating neurotransmission, *etc.* However, when an inflammatory reaction occurs, the expression of iNOS in the related cells dramatically increases, thus producing a large amount of NO. Excessively produced NO induces tissue damage, gene mutation, nerve damage, *etc.,* and increases vascular permeability to promote inflammatory reactions such as edema. As such, when the inflammatory reaction is promoted, an inflammatory disease, in which an excessive inflammatory reaction appears as a major lesion, occurs and such inflammatory diseases may occur in any part of the body.

In particular, arthritis, in which inflammation and pain appear in a joint, can be classified into osteoarthritis, rheumatoid arthritis, gout, psoriatic arthritis, *etc.* It is known that 65% of patients with arthritis suffer from osteoarthritis. According to the statistics of the Ministry of Health and Welfare of Korea, the prevalence of osteoarthritis in adults is 24.2% in those over 50 years old, 31% in those over 60 years old, and about 42% in those over 70 years old. Due to rapidly aging population, the number of patients with osteoarthritis is expected to increase rapidly, and the market size of osteoarthritis treatment in seven major countries (the USA, France, Germany, Italy, Spain, the UK, and Japan) is expected to increase from 4.3 billion USD in 2017 to 9.5 billion USD in 2024.

Accordingly, various studies for the treatment of osteoarthritis have been conducted. Treatment in early-stage osteoarthritis is mainly based on conservative measures such as protection, rest, ice packs, compression, and elevation; treatment in mid-stage osteoarthritis is mainly based on drug therapy using glucosamine, chondroitin sulfate, analgesics, non-steroidal anti-inflammatory drugs, steroids, *etc*.; and treatment in late-stage osteoarthritis is mainly based on surgery.

Research is being conducted intensively to develop various therapeutic agents for the treatment of mid-stage osteoarthritis based on drug therapy. For example, Korean Patent No. 0229343 discloses a therapeutic agent for osteoarthritis containing celecoxib, a type of non-steroidal anti-inflammatory drug (NSAIDs), as an active ingredient, and Korean Patent No. 0494265 discloses a therapeutic agent for osteoarthritis containing stem cells as an active ingredient. However, in the case of NSAIDs, there is a problem in that these temporarily alleviate symptoms and do not delay the progression of osteoarthritis or restore osteoarthritis-induced cartilage damage. In addition, in the case of stem cell-based therapy, not only are considerable time, effort and cost required for preparation and surgical procedure, but also, therapeutic efficacy is often compromised due to early cell death after injection into the body, and the risk of infection and safety issues are emerging.

### [Disclosure]

### [Technical Problem]

The present inventors have made intensive efforts to develop a treatment method that can restore damaged cartilage, which is the cause of osteoarthritis, and reduce costs and time, and as a result, they have confirmed that when a preparation containing components derived from the culture supernatant of chondrocytes is used as an active ingredient, damaged cartilage can be effectively restored, and these components derived from the culture supernatant of chondrocytes can enhance the treatment efficiency through a PCA (Precipitation with Compressed Fluid Anti-solvent) drying process, thereby completing the present invention.

### [Technical Solution]

One object of the present invention is to provide a method for preparing a culture medium-derived component or a crystallized culture medium using a PCA (Precipitation with Compressed Fluid Anti-solvent) drying process.

Another object of the present invention is to provide a pharmaceutical composition for preventing or treating osteoarthritis, including a culture medium-derived component or a crystallized culture medium prepared by way of the above method, as an active ingredient.

Still another object of the present invention is to provide a method for restoring chondrocytes using the culture medium-derived component or the crystallized culture medium prepared by way of the above method.

Yet another object of the present invention is to provide a method for treating a subject having osteoarthritis by way of the method for restoring chondrocytes using the culture medium-derived component or the crystallized culture medium.

Even another object of the present invention is to provide a method for treating a subject having osteoarthritis using the culture medium-derived component or a crystallized culture medium.

Further another object of the present invention is to provide the use of the culture medium-derived component or the crystallized culture medium for the preparation of a formulation for osteoarthritis treatment.

### [Advantageous Effects]

The culture medium-derived component provided in the present invention has excellent activity of alleviating inflammatory environment and inhibiting expression of catabolism-related genes, and the culture medium-derived component can be produced in a higher yield by way of the method provided in the present invention, and thus can be widely used in the development of therapeutic agents for osteoarthritis.

### [Brief Description of Drawings]

FIG. 1a is a graph showing the results of comparing the expression levels of inflammation-related genes (IL-1β, IL6, and iNOS) expressed in chondrocytes of four types of cell cultures (control group, No Secretome, LYO, and PCA).
FIG. 1b is a fluorescence microscopic image showing the results of immunofluorescence staining assay to confirm the expression level of iNOS protein in each of chondrocytes from four types of cell cultures (control group, No Secretome, LYO, and PCA).
FIG. 1c is a graph showing the result of analyzing the ratio of cells, in which iNOS protein is expressed, in the fluorescence micrograph.
FIG. 1d is a graph showing the result of comparing the concentration of NO contained in each culture supernatant obtained from four types of cell cultures (control, No Secretome, LYO, and PCA).
FIG. 2a is a graph showing the results of comparing the expression levels of MMP genes (MMP13, MMP3, and MMP1) expressed in chondrocytes of four types of cell cultures (control group, No Secretome, LYO, and PCA).
FIG. 2b is a graph showing the results of comparing the expression levels of ADAMTS genes (ADAMTS4 and ADAMTS5) expressed in chondrocytes of four types of cell cultures (control group, No Secretome, LYO, and PCA).
FIG. 2c shows a fluorescence micrograph showing the results of immunofluorescence staining assay to confirm the expression level of MMP13 in each chondrocyte from four types of cell cultures (control group, No Secretome, LYO, and PCA), and a graph showing the results of analyzing the ratio of the average fluorescence intensity of MMP13 protein shown in the fluorescence micrograph.
FIG. 3a is a micrograph showing the results of tissue staining for four types of joints (Normal, PBS, LYO, and PCA).
FIG. 3b is a graph showing the results of comparing Modified Mankin's Scores evaluated for four types of joints (Normal, PBS, LYO, and PCA).
FIG. 3c is a fluorescence micrograph showing the results of immunofluorescence staining assay to confirm the expression levels of osteoarthritis-related proteins (MMP13, NITEGE, and iNOS) in four types of joints (Normal, PBS, LYO, and PCA).
FIG. 3d is a graph showing the result of measuring the ratio of cells, in which MMP13 is expressed, in the fluorescence micrograph.
FIG. 3e is a graph showing the result of measuring the ratio of cells, in which NITEGE is expressed, in the fluorescence micrograph.
FIG. 3f is a graph showing the result of measuring the ratio of cells, in which iNOS is expressed, in the fluorescence micrograph.
FIG. 4a is a graph showing the results of comparing the change in yield of culture medium-derived components of chondrocytes according to the mixing ratio of DMSO and acetone, in performing PCA drying process for the culture medium of chondrocytes.
FIG. 4b is a graph showing the results of comparing the change in yield of culture medium-derived components of chondrocytes according to the mixing ratio of co-solvent, in which the mixing ratio of DMSO and acetone is 1.5:1 (v/v), and the culture medium of chondrocytes, in performing PCA drying process for the culture medium of chondrocytes.
FIG. 4c is a graph showing the results of comparing the change in yield of culture medium-derived components of chondrocytes according to the mixing ratio of co-solvent, in which the mixing ratio of DMSO and acetone is 1.1:1 (v/v), and the culture medium of chondrocytes, in performing PCA drying process for the culture medium of chondrocytes.
FIG. 4d is a graph showing the results of comparing the change in yield of culture medium-derived components of chondrocytes according to the temperature conditions when using a co-solvent, in which the mixing ratio of DMSO and acetone is 1.5:1 (v/v), in performing the PCA drying process for the culture medium of chondrocytes.
FIG. 4e is a graph showing the results of comparing the change in yield of culture medium-derived components of chondrocytes according to the temperature conditions when using a co-solvent, in which the mixing ratio of DMSO and acetone is 1:1 (v/v), in performing the PCA drying process for the culture medium of chondrocytes.
FIG. 4f is a graph showing the results of comparing the change in yield of culture medium-derived components of chondrocytes according to the pressure conditions when using a co-solvent, in which the mixing ratio of DMSO and acetone is 1.5:1 (v/v), in performing the PCA drying process for the culture medium of chondrocytes.
FIG. 4g is a graph showing the results of comparing the change in yield of culture medium-derived components of chondrocytes in log scale according to the pressure conditions when using a co-solvent, in which the mixing ratio of DMSO and acetone is 1.5:1 (v/v), in performing the PCA drying process for the culture medium of chondrocytes.

### [Detailed Description of Preferred Embodiments]

In order to achieve the objects described above, one aspect of the present invention provides a method for preparing a culture medium-derived component or a crystallized culture medium.

Specifically, the method for preparing a culture medium-derived component or a crystallized culture medium of the present invention includes:
(a) adding a co-solvent containing DMSO and acetone to a culture medium to obtain a mixture;
(b) adding a compressed anti-solvent to the mixture obtained above; and
(c) precipitating the culture medium-derived components in a pressure vessel.

The culture medium-derived component or the crystallized culture medium provided in the present invention can be finally obtained by culturing desired cells, obtaining the culture supernatant, and performing a PCA (Precipitation with Compressed Fluid Anti-solvent) drying process on the obtained culture supernatant.

As used herein, the term "PCA (Precipitation with Compressed Fluid Anti-solvent) drying process" is defined as a technology in which a desired component is precipitated and crystallized using a compressed liquid or supercritical anti-solvent. Roughly, the process includes the steps of supplying a culture medium containing a first solvent and a target material dissolved in the first solvent to a container, supplying a third solvent to the container, and supplying a second solvent to the container, wherein the third solvent serves to make the two phases of the first solvent and the second solvent into a single phase, and the target material dissolved in the first solvent is precipitated and then dried or crystallized by performing the above steps. Such PCA drying processes are known in the art (Korean Patent Application Publication No. 10-2020-0106779, Korean Patent No. 10-2154923)

In comparison of the method for preparing a culture medium-derived component or a crystallized culture medium provided in the present invention with the known PCA drying process, the culture supernatant corresponds to the first solvent, the co-solvent corresponds to the third solvent, and the compressed anti-solvent corresponds to the second solvent.

First, the culture medium provided in the present invention may have the same meaning as the culture supernatant, and roughly means a liquid component in a state in which cells have been removed from the cell culture by incubating cell to obtain a culture medium, and then subjecting the culture medium to methods such as centrifugation, filtration, *etc.*

The cells to be incubated to obtain the culture supernatant are not particularly limited to, but may be, for example, mesenchymal stem cells, connective tissue-derived cells, *etc.,* and as another example, the cells may be fibrous connective tissue-derived cells, as still another example, they may be cells derived from cartilage tissue, as yet another example, they may be chondrocytes, as even another example, they may be chondrocytes incubated for at least 3 days, and as further another example, they may be chondrocytes incubated for at least one week.

In addition, the medium for culturing the cells is not particularly limited to, but as an example, a medium containing growth factors, extracellular matrix (ECM), extracellular vesicles, chemokines, cytokines, *etc.*

Next, the co-solvent provided in the present invention is also not particularly limited, but as an example, ethanol, ethyl acetate, butanol, isopropyl alcohol, DMSO, acetone, *etc.* may be used individually or in combination, as another example, it may be a mixed solvent containing DMSO and acetone, as still another example, it may be a mixed solvent containing DMSO and acetone in a ratio of 2:1, 1.9:1, 1.8:1, 1.7:1, 1.6:1, 1.5:1, 1.4:1, 1.3:1, 1.2:1, 1.1:1, or 1:1 (v/v), and as yet another example, it may be a mixed solvent containing DMSO and acetone in a ratio of 1.5:1, 1.4:1, 1.3:1, 1.2:1, 1.1:1, or 1:1 (v/v).

In some cases, when the mixed solvent containing DMSO and acetone is used as a co-solvent, a solvent of alcohol component such as ethanol, ethyl acetate, butanol, or isopropyl alcohol, *etc.* may be further included. In particular, the content of the alcohol component solvent added is not particularly limited, but as an example, it may be 0.1% (v/v), 0.2% (v/v), 0.3% (v/v), 0.4% (v/v), 0.5% (v/v), 0.6% (v/v), 0.7% (v/v), 0.8% (v/v), 0.9% (v/v), 1.0% (v/v), 1.1% (v/v), 1.2% (v/v), 1.3% (v/v), 1.4% (v/v), 1.5% (v/v), 1.6% (v/v), 1.7% (v/v), 1.8% (v/v), 1.9% (v/v), 2.0% (v/v), 2.1% (v/v), 2.2% (v/v), 2.3% (v/v), 2.4% (v/v), 2.5% (v/v), 2.6% (v/v), 2.7% (v/v), 2.8% (v/v), 2.9% (v/v), 3.0% (v/v), 3.1% (v/v), 3.2% (v/v), 3.3% (v/v), 3.4% (v/v), 3.5% (v/v), 3.6% (v/v), 3.7% (v/v), 3.8% (v/v), 3.9% (v/v), 4.0% (v/v), 4.1% (v/v), 4.2% (v/v), 4.3% (v/v); 4.4% (v/v), 4.5% (v/v), 4.6% (v/v), 4.7% (v/v), 4.8% (v/v), 4.9% (v/v), or 5% (v/v), as another example, it may be 0.5% (v/v), 0.6% (v/v), 0.7% (v/v), 0.8% (v/v), 0.9% (v/v), 1.0% (v/v), 1.1% (v/v), 1.2% (v/v), 1.3% (v/v), 1.4% (v/v), 1.5% (v/v), 1.6% (v/v), 1.7% (v/v), 1.8% (v/v), 1.9% (v/v), 2.0% (v/v), 2.1% (v/v), 2.2% (v/v), 2.3% (v/v), 2.4% (v/v), 2.5% (v/v), 2.6% (v/v), 2.7% (v/v), 2.8% (v/v), 2.9% (v/v), or 3% (v/v); and as still another example, it may be 1% (v/v).

Lastly, the compressed anti-solvent provided in the present invention is not particularly limited, but as an example, it may be carbon dioxide, dimethyl ether, N₂O, *etc.,* as another example, it may be carbon dioxide, and as still another example, it may be supercritical carbon dioxide.

Meanwhile, the co-solvent is mixed with the culture medium to form a mixture, and the mixing ratio of the co-solvent and the culture medium is not particularly limited, but for example, the co-solvent and the culture medium may be mixed in a ratio of at least 18:1 (v/v), s another example, the co-solvent and the culture medium may be mixed in a ratio of 25:1, 26:1, 27:1, 28:1, 29:1, 30:1, 31:1, 32:1, 33:1, 34:1, 35:1, 36:1, or 37:1 (v/v), as still another example, the co-solvent and the culture medium may be mixed in a ratio of 30:1, 31:1, 32:1, 33:1, 34:1, or 35:1 (v/v).

When a compressed anti-solvent is added to the mixture of the co-solvent and the culture medium and a certain range of temperature and pressure conditions are applied, the solvent component contained in the culture medium is replaced with the compressed anti-solvent. Since the substituted compressed anti-solvent does not show solubility with respect to components other than the solvent component contained in the culture medium, the components other than the solvent component are precipitated. When the maintained temperature and pressure are changed to room temperature and atmospheric pressure conditions after precipitation of the components other than the solvent component, the anti-solvent component loses the characteristics of the solvent and changes to a gaseous form, and thus, the precipitated components other than the solvent component can be obtained in a dried form, and accordingly, the thus-obtained dried product may be called a culture medium-derived component or a crystallized culture medium.

When performing precipitation by adding a compressed anti-solvent to the mixture of the co-solvent and the culture medium, the temperature condition is not particularly limited, but as an example, it may be 15°C, 16°C, 17°C, 18°C, 19°C, 20°C, 21 °C, 22°C, 23°C, 24°C, 25°C, 26°C, 27°C, 28°C, 29°C, or 30°C, as another example, it may be 20°C, 21°C, 22°C, 23°C, 24°C, or 25°C, and as still another example, it may be 20°C.

When performing precipitation by adding a compressed anti-solvent to the mixture of the co-solvent and the culture medium, the pressure condition is not particularly limited, but as an example, it may be 70 bar, 80 bar, 90 bar, 100 bar, 110 bar, 120 bar, 130 bar, 140 bar, 150 bar, 160 bar, 170 bar, 180 bar, 190 bar, or 200 bar, as another example, it may be 125 bar, and as still another example, it may be 200 bar.

According to one embodiment of the present invention, the culture supernatant obtained by culturing chondrocytes was subjected to a freeze-drying method or a PCA drying process to obtain each dried product, and the chondrocyte recovery effect of each dried product, was measured. As a result, it was confirmed that the dried product obtained by the PCA drying process exhibited a significantly higher level of chondrocyte recovery effect than the dried product obtained by the freeze-drying method. In addition, as a result of administering each of the dried products to the osteoarthritis model animal, it was confirmed that the dried product obtained by the PCA drying process exhibited a significantly higher level of osteoarthritis treatment effect than the dried product obtained by the freeze-drying method.

Another aspect of the present invention provides a pharmaceutical composition for preventing or treating osteoarthritis, including the culture medium-derived component or the crystallized culture medium prepared by way of the method.

As described above, the culture medium-derived component or crystallized culture medium obtained by applying the culture supernatant obtained by culturing chondrocytes to the PCA drying process can exhibit a chondrocyte recovery effect and an osteoarthritis treatment effect, and accordingly, the culture medium-derived component or crystallized culture medium may be used as an active ingredient in preparations for the prevention or treatment of osteoarthritis.

As used herein, the term "osteoarthritis", also referred to as degenerative arthritis, means a kind of inflammatory disease in which the cartilage area present in a joint is damaged and exhibits inflammation, swelling, *etc.*

As used herein, the term "inflammatory disease" refers to a disease caused by proinflammatory substances (inflammatory cytokines) such as tumor necrosis factor-α (TNF-α), interleukin-1 (IL-1), IL-6, prostaglandin, leukotriene, or nitric oxide (NO), which are secreted from immune cells such as macrophages by excessively enhancing the human immune system due to harmful stimuli such as proinflammatory factors or irradiation.

Typically, destruction of articular cartilage tissues in osteoarthritis basically begins with the loss of homeostasis in articular cartilage tissues. In normal joints, physicochemical or kinetic pathways involved in the synthesis of ECM materials and maintenance of homeostasis are in balance, so that the chondrocytes synthesize cartilage-specific ECM proteins such as sulfated proteoglycan (aggrecan) and type II collagen. However, the homeostasis of cartilage ECM synthesis and degradation is first disrupted by synthesis and activation of matrix metalloproteinase (MMP) in cartilage tissues of diseases such as degenerative arthritis. In osteoarthritis tissues, the expression and activity of collagenase-1 (MMP-1), MMP-2, stromelysin-1 (MMP-3), neutrophil collagenase (MMP-8), gelatinases (MMP-9), collagenase-3 (MMP-13), and matrilysin (MMP-7) having a wide range of substrate specificity, *etc.,* increase, and this phenomenon is induced by inflammatory cytokines such as IL-1β and tumor necrosis factor (TNF)-α. Preservation of cartilage tissues depends on auxiliary proteins such as type 2 collagen, proteoglycan, and fibronectin, and these molecules are synthesized in chondrocytes to form tissues. In particular, aggrecan plays the most important role among intra-articular proteoglycans, and is particularly sensitive to stromelysin-1, in which the interglobular domain (IGD) of aggrecan is activated, and MMP-8 and MMP-13 having the functions of aggrecan, degrade IGD of aggrecan. Among A Disintegrin and Metalloproteinase with Thrombospondin Motifs (ADAMTS), ADAMTS4 and ADAMTS5 have the function of aggrecanase. The degradation of aggrecan is important for the destruction of cartilage tissues, but the ultimate destruction of joint tissue is caused by the loss of cartilage collagen. Accordingly, MMP-1, which degrades the helical domain of type 2 collagen, and stromelysin (MMP-3), which degrades type IX and type XI collagen, are involved in cartilage tissue destruction. In addition, MMPs in chondrocytes and synovial cells are synthesized as latent pro-enzymes and activated during the OA process. Therefore, in order to control the degeneration of cartilage tissues, the activity of MMPs can be inhibited, thereby inhibiting the degradation of joint ECM.

As used herein, the term "prevention" refers to all kinds of actions that inhibit or delay osteoarthritis by administration of the pharmaceutical composition.

As used herein, "treatment" refers to all kinds of actions of clinical intervention in order to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. For the purpose of the present invention, the treatment may be interpreted as including all kinds of actions in which symptoms of osteoarthritis are alleviated or cured by administration of the pharmaceutical composition, but is not particularly limited thereto.

The pharmaceutical composition may further contain an appropriate carrier, excipient, or diluent which is conventionally used in the preparation of pharmaceutical compositions, and the carrier may be a non-natural carrier. Examples of the carrier, excipient, or diluent may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.*

Meanwhile, the pharmaceutical composition of the present invention may be formulated in the form of powders, granules, tablets, capsules, suspensions, emulsions, syrups, aerosols, *etc.* for oral administration, agents for external use, suppositories, and sterile injection solutions according to each conventional method. The formulations may be prepared using a commonly used diluent or excipient such as a filler, extender, binder, humectant, disintegrant, surfactant, *etc.* Solid formulations for oral administration may include tablets, pills, powders, granules, capsules, *etc.,* and these solid formulations may be prepared by adding at least one excipient (*e*.*g*., starch, calcium carbonate, sucrose or lactose, gelatin, *etc*.). Additionally, lubricants such as magnesium stearate, talc, *etc.* may be used in addition to the simple excipient. Liquid formulations for oral administration may include suspensions, liquid medicine for internal use, emulsions, syrups, *etc.,* and various excipients such as humectants, sweeteners, fragrances, preservatives, *etc.* may be used in addition to the simple diluents such as water and liquid paraffin. Formulations for parenteral administration may include sterile aqueous solutions, non-aqueous solvents, suspensions, emulsions, lyophilized formulations, suppositories, *etc.* Examples of the non-aqueous solvents and suspensions may include propylene glycol, polyethylene glycol, and vegetable oils such as olive oil; an injectable ester such as ethyl oleate, *etc.* Examples of the bases for suppositories may include Witepsol, macrogol, Tween 61, cacao butter, laurinum, glycerogelatin, *etc.*

The pharmaceutical composition may be prepared in any formulation selected from the group consisting of tablets, pills, powders, granules, capsules, suspensions, liquid medicine for internal use, emulsions, syrups, sterile injection solutions, non-aqueous solvents, lyophilized formulations, and suppositories.

The content of the culture medium-derived component or the crystallized culture medium contained in the pharmaceutical composition of the present invention is not particularly limited, but may be contained in an amount of 0.0001%, 0.001%, 0.01%, 0.05%, 0.1%, 0.2%, 0.3%, 0.4%, 0.5%, 0.6%, 0.7%, 0.8%, 0.9%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, or 50% by weight based on the total weight of the final composition, preferably 0.001%, 0.01%, 0.05%, 0.1%, 0.2%, 0.3%, 0.4%, 0.5%, 0.6%, 0.7%, 0.8%, 0.9%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, or 10% by weight.

The pharmaceutical composition of the present invention may be administered in a pharmaceutically effective amount. As used herein, the term "pharmaceutically effective amount" refers to an amount sufficient for the treatment of diseases at a reasonable benefit/risk ratio applicable to medical treatment, and the level of the effective dose may be determined based on the factors including the kind of a subject, severity of illness, age, sex, drug activity, drug sensitivity, duration of administration, administration route and dissolution rate, duration of treatment, factors including drugs to be used simultaneously in combination, and other factors well known in the medical field. The pharmaceutical composition of the present invention may be administered as an individual therapeutic agent, in combination with other therapeutic agents, or sequentially or simultaneously with conventional therapeutic agents, and may be administered once or multiple times. It is important to administer the pharmaceutical composition in an amount to obtain the maximum effect with a minimum amount without adverse effects considering all of the factors described above.

The dose of the pharmaceutical composition of the present invention can be determined by one of ordinary skill in the art in consideration of the purpose of use, addicted level of a disease, age, weight, sex, and medical history of a patient, kinds of materials used as active ingredients, *etc.* For example, the pharmaceutical composition of the present invention may be administered in an amount of about 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, 25 mg/kg, 26 mg/kg, 27 mg/kg, 28 mg/kg, 29 mg/kg, 30 mg/kg, 31 mg/kg, 32 mg/kg, 33 mg/kg, 34 mg/kg, 35 mg/kg, 36 mg/kg, 37 mg/kg, 38 mg/kg, 39 mg/kg, 40 mg/kg, 45 mg/kg, 50 mg/kg, 55 mg/kg, 60 mg/kg, 65 mg/kg, 70 mg/kg, 75 mg/kg, 80 mg/kg, 85 mg/kg, 90 mg/kg, 95 mg/kg, or 100 mg/kg for an adult, and preferably 1 mg/kg, 2 mg/kg, 3 mg/kg, 4 mg/kg, 5 mg/kg, 6 mg/kg, 7 mg/kg, 8 mg/kg, 9 mg/kg, or 10 mg/kg. The pharmaceutical composition of the present invention may be administered once a month or in a few divided doses per month, but the frequency of administration is not particularly limited thereto.

Still another aspect of the present invention provides a method for restoring chondrocytes using the culture medium-derived component or the crystallized culture medium.

As described above, since it was confirmed that the culture medium-derived component or the crystallized culture medium prepared by way of the above method exhibits a significantly higher level of cartilage recovery effect than the dried product obtained by the freeze-drying method, when damaged chondrocytes or cartilage tissues are treated with the culture medium-derived component or the crystallized culture medium, the damaged chondrocytes or cartilage tissues can be restored.

Yet another aspect of the present invention provides a method for treating a subject having osteoarthritis by way of the method for restoring chondrocytes using the culture medium-derived component or the crystallized culture medium.

In other words, the present invention provides a method for preventing or treating osteoarthritis, including: administering the culture medium-derived component or the crystallized culture medium to a subject except human who has or is likely to develop osteoarthritis.

In particular, the definitions of the culture medium-derived component or crystallized culture medium, osteoarthritis, prevention and treatment are as described above.

As used herein, the term "subject" means any animal that has the risk of developing osteoarthritis or includes a tissue with the disease, but humans are excluded. By administering the culture medium-derived component or the crystallized culture medium of the present invention to a subject, osteoarthritis can be alleviated or treated.

As used herein, the term "alleviation" refers to all kinds of actions that improve or advantageously change osteoarthritis by administration of the culture medium-derived component or the crystallized culture medium according to the present invention.

As used herein, the term "treatment" refers to all kinds of actions in which the treatment of osteoarthritis carried out or which advantageously change the treatment of osteoarthritis by administration of the culture medium-derived component or the crystallized culture medium according to the present invention to a subject in need of osteoarthritis treatment.

The culture medium-derived component or the crystallized culture medium of the present invention, or the pharmaceutical composition containing the same is administered in a pharmaceutically effective amount.

As used herein, the term "administration" refers to the introduction of the culture medium-derived component or the crystallized culture medium, or the pharmaceutical composition containing the same to a subject by any appropriate method. The administration may be carried out via various various oral or parenteral routes as long as a target tissue can be reached.

The culture medium-derived component or the crystallized culture medium of the present invention, or the pharmaceutical composition containing the same may be appropriately administered to a subject according to the conventional method, route of administration, and dose used in the art depending on the purpose or necessity. Examples of the administration routes may include oral, parenteral, subcutaneous, intraperitoneal, intrapulmonary, and intranasal administrations, and the parenteral administration may include intramuscular, intravenous, intraarterial, intraperitoneal, and subcutaneous administrations. Additionally, an appropriate dose and the frequency of administration may be selected according to the methods known in the art, and the dose and the frequency of administration of the culture medium-derived component or the crystallized culture medium of the present invention, or the pharmaceutical composition containing the same actually administered may be appropriately determined by various factors such as the type of the symptom to be treated, administration route, sex, health conditions, diet, age and weight of a subject, and severity of the disease.

As used herein, the term "pharmaceutically effective amount" refers to an amount sufficient for the inhibition or alleviation of osteoarthritis at a reasonable benefit/risk ratio applicable to medical treatment, and the level of the effective dose may be determined based on the factors including the kind of a subject, severity of illness, age, sex, drug activity, drug sensitivity, duration of administration, administration route and dissolution rate, duration of treatment, factors including drugs to be used simultaneously in combination, and other factors well known in the medical field. The culture medium-derived component or the crystallized culture medium of the present invention, or the pharmaceutical composition containing the same may be administered as an individual therapeutic agent, in combination with other therapeutic agents, or sequentially or simultaneously with conventional therapeutic agents, and may be administered once or multiple times. It is important to administer the pharmaceutical composition in an amount to obtain the maximum effect with a minimum amount and without adverse effects considering all of the factors described above, and the pharmaceutically effective amount can easily be determined by one of ordinary skill in the art.

Even another aspect of the present invention provides the use of the culture medium-derived component or the crystallized culture medium for the preparation of a formulation for osteoarthritis treatment.

### [Mode for Carrying Out the Invention]

Hereinafter, the present invention will be described in more detail with reference to the following Examples. However, these Examples are for illustrative purposes only and the scope of the invention is not limited by these Examples.

### Example 1: Preparation of Culture Medium-Derived Component of Chondrocytes

### Example 1-1: Cultivation of Chondrocytes

Cartilage fragments obtained from cartilage tissues were treated with a 0.15% (w/v) type II collagenase (Worthington Biochemical) solution at 37°C for 18 hours. Then, a cell solution, from which residues were removed, was obtained using a 70 µm cell strainer, and subsequently, the obtained cell solution was centrifuged (1100 rpm, 5 minutes) to obtain precipitated chondrocytes.

Next, the obtained chondrocytes were resuspended into a medium for culturing chondrocytes at a concentration of 10,000 cells/cm² to 40,000 cells/cm², and incubated at 37°C and 5% CO₂ in such a manner that the medium was replaced every 3 days. In particular, the composition of the medium for culturing chondrocytes used is as follows: 10% (v/v) fetal bovine serum (FBS; Corning), 1% (v/v) penicillin/streptomycin (Gibco), 1% (v/v) non-essential amino acids (NEAA, Gibco), 1% (v/v) 4-(2-hydroxyethyl)-1-piperazineethanesulfonic acid (HEPES, Gibco), 8 µg/mL L-proline (Sigma), and 20 µg/mL L-ascorbic acid (Sigma)

The chondrocytes were cultured until the saturation of the chondrocytes reached 100%, and then, the medium was removed, and the chondrocytes were washed twice with phosphate buffer. Subsequently, the culture medium for chondrocytes, from which FBS was removed, was added to the chondrocytes again and incubated for 7 days, and a culture supernatant was obtained therefrom.

### Example 1-2: Preparation of Culture Medium-Derived Component of Chondrocytes Using Freeze-Drying

The culture supernatant obtained in Example 1-1 was frozen in a -80°C freezer for 1 day, and then freeze-dried for 3 days at -40°C and 0.08 mbar using a freeze dryer (Labconco, cat. # 7670540) to obtain culture-medium components of chondrocytes prepared via freeze-drying.

### Example 1-3: Preparation of Culture Medium-Derived Component of Chondrocytes Using PCA Dyring

First, carbon dioxide in a supercooled liquid state was supplied at a flow rate of 100 mL/min to a reactor of the PCA process in which the temperature and pressure were maintained at 20°C and 200 bar.

Next, a solution, in which a co-solvent, in which DMSO and acetone were mixed at a volume ratio of 1.5:1, and the culture supernatant obtained in Example 1-1 were mixed at 30:1 (v/v) was sprayed at a flow rate of 1 mL/min to the reactor to produce a multi-component mixture containing water, carbon dioxide, and acetone/DMSO inside the reactor. Then, the culture medium-derived components of chondrocytes were precipitated therefrom.

Finally, after the components were precipitated, carbon dioxide was additionally supplied for 1 hour to remove the remaining culture supernatant components and co-solvent, and the reactor was reduced under pressure to vaporize carbon dioxide. Thereafter, the precipitated culture medium-derived components of chondrocytes were obtained through a filter.

### Example 2: Efficacy Analysis of Culture Medium-Derived Components of Chondrocytes

### Example 2-1: In Vitro Assay

### Example 2-1-1: Preparation of Sample

Chondrocytes cultured by way of the method of Example 1-1 were treated with IL-1β and incubated for 24 hours to prepare chondrocytes in which an inflammatory response was induced. In particular, as a control, chondrocytes cultured without treatment with IL-1β were used.

The thus-prepared chondrocytes, in which the inflammatory response was induced, were treated with in three groups of media for 3 days to obtain respective cell cultures. The three types of media include: a medium without containing the culture medium-derived components of chondrocytes (No Secretome); a medium containing 1 mg/mL of the culture medium-derived components of chondrocytes prepared in Example 1-2 using freeze-drying (LYO); and a medium containing 1 mg/mL of the culture medium-derived components of chondrocytes prepared in Example 1-3 using PCA drying (PCA), which were used receptively.

### Example 2-1-2: Expression Level Analysis of Genes Related to Inflammatory Response

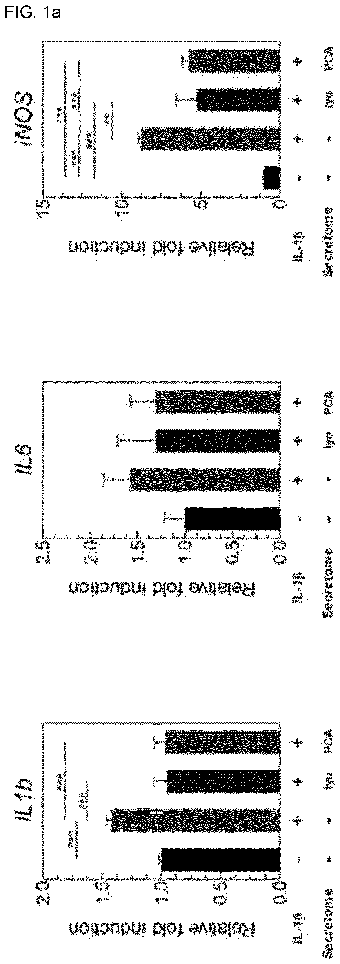

Respective chondrocytes were obtained from the four types of cell cultures (control, No Secretome, LYO, and PCA) prepared in Example 2-1-1, and the expression levels of inflammation-related gene (IL- 1β, IL6, and iNOS) expressed in each of the obtained chondrocytes were compared (FIG. 1a).

FIG. 1a is a graph showing the results of comparing the expression levels of inflammation-related genes (IL-1β, IL6, and iNOS) expressed in chondrocytes of four types of cell cultures (control group, No Secretome, LYO, and PCA).

As shown in FIG. 1a, it was confirmed that the expression levels of all three inflammation-related genes (IL-1β, IL6, and iNOS) were increased in the chondrocytes, in which the inflammatory response was induced (No Secretome), compared to the control group, but were decreased in the chondrocytes incubated in the media containing the culture medium-derived components of chondrocytes (LYO and PCA) compared to the chondrocytes in which the inflammatory response was induced (No Secretome).

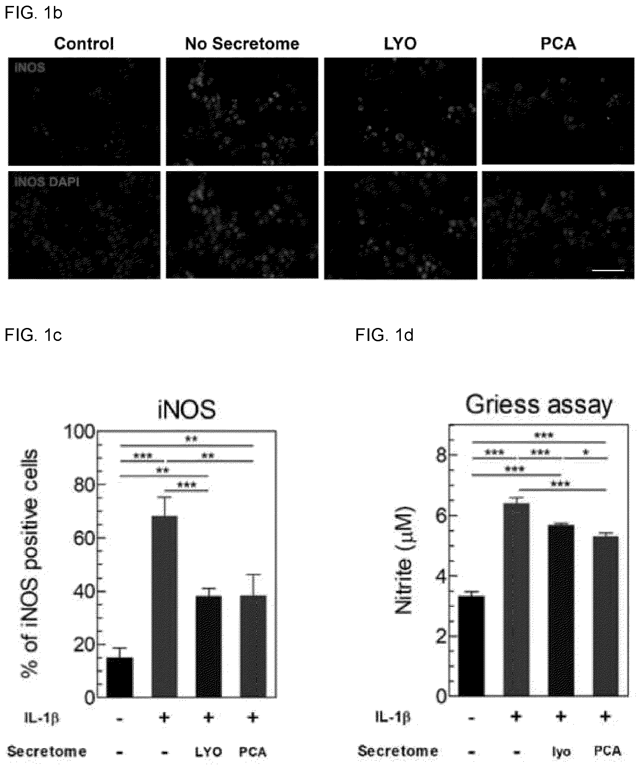

In order to verify the above results, an immunofluorescence staining assay was performed to confirm the expression level of iNOS in each of chondrocytes from four types of cell cultures (control group, No Secretome, LYO, and PCA) (FIGS. 1b and 1c).

FIG. 1b is a fluorescence micrograph showing the results of immunofluorescence staining assay to confirm the expression level of iNOS protein in each of chondrocytes from four types of cell cultures (control group, No Secretome, LYO, and PCA), and FIG. 1c is a graph showing the results of analyzing the ratio of cells, in which iNOS protein was expressed, in the fluorescence micrograph.

As shown in FIGS. 1b and 1c, the expression level of the iNOS protein was increased in the chondrocytes, in which the inflammatory response was induced (No Secretome), compared to the control group, but was decreased in the chondrocytes incubated in the media containing the culture medium-derived components of chondrocytes (LYO and PCA) compared to the chondrocytes in which the inflammatory response was induced (No Secretome).

### Example 2-1-3: Analysis of NO Levels Produced from Chondrocytes

Griess assay was performed to confirm the level of NO production in the chondrocytes in which the inflammatory response was induced.

Roughly, each of the culture supernatants from the four types of cell cultures (control, No Secretome, LYO, and PCA) prepared in Example 2-1-1 was applied to the Griess Assay Kit (Sigma, cat # MAK367). That is, 100 µL of a 10-fold diluted solution of each culture supernatant, 10 µL of Griess Reagent I, 10 µL of Griess Reagent II, and 80 µL of nitrite assay buffer were added to each well of a 96-well plate and reacted at room temperature for 10 minutes, and then absorbance (A540) was measured at 540 nm.

In addition, a standard curve was prepared by performing a Griess assay based on the known NO concentration solution, and then the concentration of NO contained in each culture supernatant was calculated by applying the measured absorbance values (FIG. 1d).

FIG. 1d is a graph showing the results of comparing the concentration of NO contained in each culture supernatant obtained from the four types of cell cultures (control, No Secretome, LYO, and PCA).

As shown in FIG. 1d, it was confirmed that the concentration of NO contained in the culture supernatant of each chondrocyte showed a significantly higher level in the chondrocytes in which the inflammatory response was induced (No Secretome), compared to the control group, but was decreased in the chondrocytes incubated in the media containing the culture medium-derived components of chondrocytes (LYO and PCA) compared to the chondrocytes in which the inflammatory response was induced (No Secretome).

In addition, among the media containing the culture medium-derived components of chondrocytes, it was confirmed that the chondrocytes incubated in the medium containing PCA-dried components showed a relatively low level of NO concentration than in the chondrocytes incubated in the medium containing freeze-dried components (LYO).

### Example 2-1-4: Analysis of Expression Level of Catabolism-Related Genes

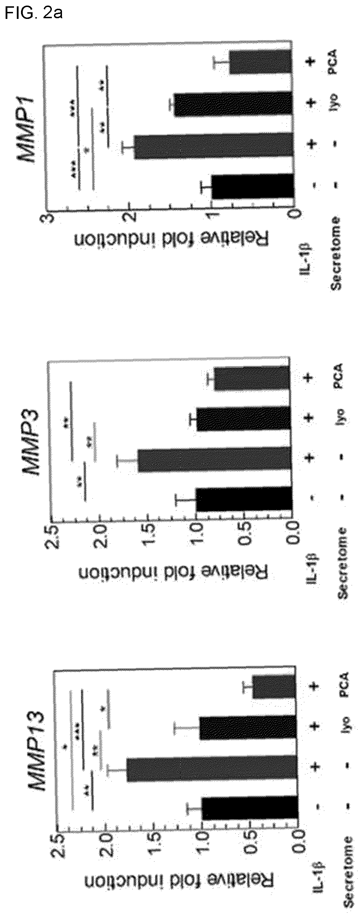

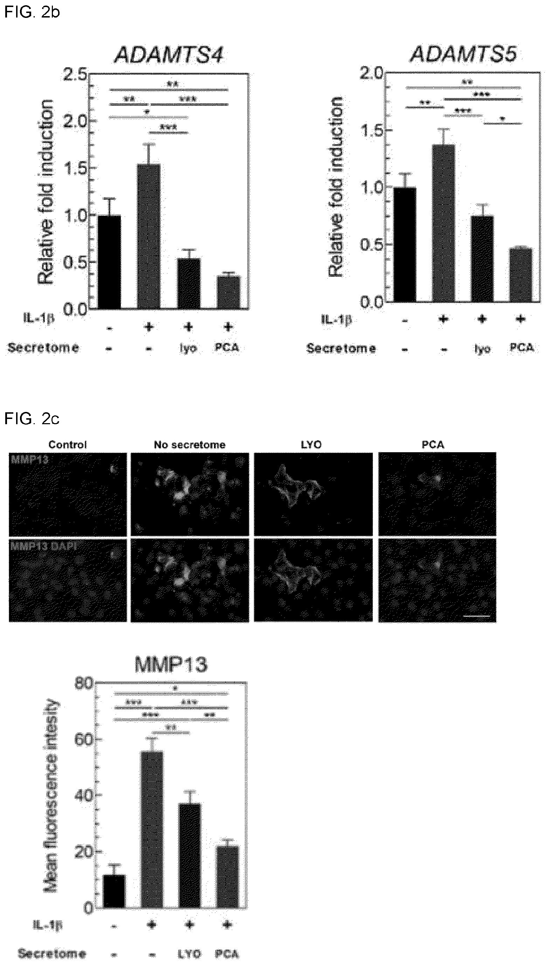

Respective chondrocytes were obtained from the four types of cell cultures (control group, No Secretome, LYO, and PCA) prepared in Example 2-1-1, and the expression levels of MMP genes (MMP13, MMP3, and MMP1) and ADAMTS genes (ADAMTS4 and ADAMTS5), which are related to catabolism expressed in each of the chondrocytes, were compared (FIGS. 2a and 2b).

FIG. 2a is a graph showing the results of comparing the expression levels of MMP genes (MMP13, MMP3, and MMP1) expressed in chondrocytes of four types of cell cultures (control group, No Secretome, LYO, and PCA).

As shown in FIG. 2a, it was confirmed that the expression levels of all three MMP genes (MMP13, MMP3, and MMP1) were increased in the chondrocytes, in which the inflammatory response was induced (No Secretome), compared to the control group, but were decreased in the chondrocytes incubated in the media containing the culture medium-derived components of chondrocytes (LYO and PCA) compared to the chondrocytes in which the inflammatory response was induced (No Secretome). In addition, three types of MMP genes in the chondrocytes incubated in the medium containing freeze-dried components (LYO), among the media containing the culture medium-derived components of chondrocytes, were expressed at a similar or slightly higher level than the control group, but MMP genes in the chondrocytes incubated in the medium containing PCA-dried components (PCA) were expressed at a lower level than the control group.

FIG. 2b is a graph showing the results of comparing the expression levels of ADAMTS genes (ADAMTS4 and ADAMTS5) expressed in chondrocytes of four types of cell cultures (control group, No Secretome, LYO, and PCA).

As shown in FIG. 2b, it was confirmed that both ADAMTS genes (ADAMTS4 and ADAMTS5) showed increased expression levels in the chondrocytes in which the inflammatory response was induced (No Secretome) compared to the control group, but showed decreased expression levels in the chondrocytes incubated in the media containing the culture medium-derived components of chondrocytes (LYO and PCA) compared to the chondrocytes in which the inflammatory response was induced (No Secretome). In addition, it was confirmed that the two types of ADAMTS genes were expressed at a relatively low level in the chondrocytes incubated in the medium containing PCA-dried components (PCA) than in the chondrocytes incubated in the medium containing freeze-dried components (LYO), among the media containing the culture medium-derived components of chondrocytes.

In order to verify the above results, an immunofluorescence staining assay was performed to confirm the expression level of MMP13 in each chondrocyte from four types of cell cultures (control group, No Secretome, LYO, and PCA) (FIG. 2c).

FIG. 2c shows a fluorescence micrograph showing the results of performing an immunofluorescence staining assay to confirm the expression level of MMP13 in each chondrocyte from four types of cell cultures (control group, No Secretome, LYO, and PCA), and a graph showing the results of analyzing the ratio of the average fluorescence intensity of the MMP13 protein shown in the fluorescence micrograph.

As shown in FIG. 2c, it was confirmed again that the expression level of MMP13 protein was increased in the chondrocytes in which the inflammatory response was induced (No Secretome) compared to the control group, but was decreased in the chondrocytes incubated in the media containing the culture medium-derived components of chondrocytes (LYO and PCA) compared to the chondrocytes in which the inflammatory response was induced (No Secretome).

### Example 2-2: In Vivo Assay

### Example 2-2-1: Induction of Osteoarthritis and Administration of Active Ingredients

The joint of rats was incised, and the meniscus in the joint was incised and removed to induce osteoarthritis. After the surgery was completed, the rats were raised for 4 weeks, and then individuals having osteoarthritis were selected, and 50 µL of the culture medium-derived components of chondrocytes was injected to the selected individuals once per joint. In particular, as a control group, rats (Normal) in which osteoarthritis was not induced were used, and as the culture medium-derived components of chondrocytes, PBS not containing the culture medium-derived components of chondrocytes (PBS); PBS in which the culture medium-derived components of chondrocytes prepared using freeze-drying in Example 1-2 were dissolved at 10 mg/mL (LYO); and PBS (PCA) in which the culture medium-derived components of chondrocytes prepared using PCA drying in Examples 1-3 were dissolved at 10 mg/mL (PCA) were used, respectively.

After injecting the culture medium-derived components of chondrocytes, respective rats were raised for 4 weeks, and the raised rats were sacrificed to extract a joint region from each rat, which was then used in the experiments.

### Example 2-2-2: Osteoarthritis Progression Assessment

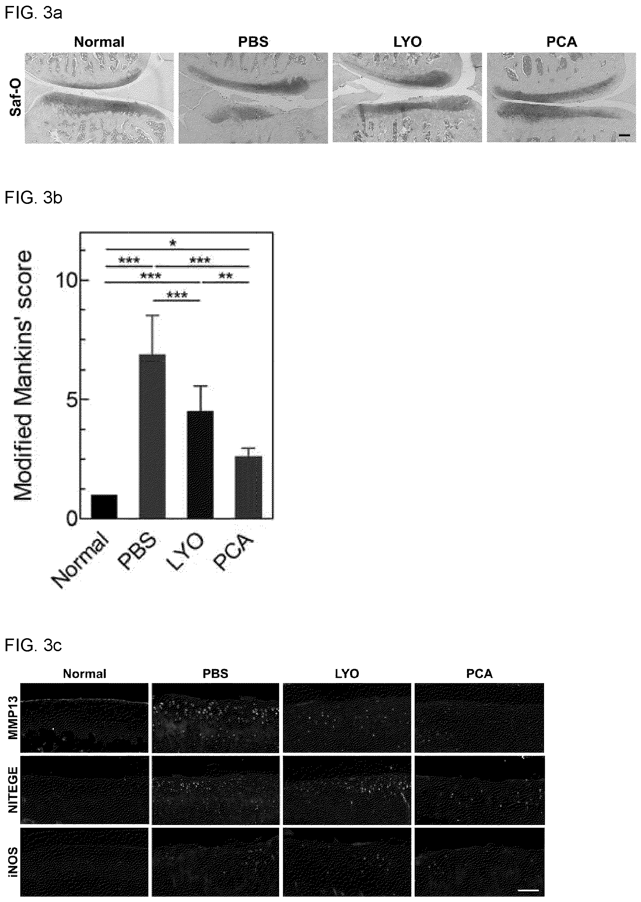

Tissue staining using Safranin-O was performed on each joint region extracted in Example 2-2-1 (FIG. 3a).

FIG. 3a is a micrograph showing the results of tissue staining for four types of joints (Normal, PBS, LYO, and PCA).

As shown in FIG. 3A, the cartilage tissue was lost in the joint (PBS) to which the culture medium-derived components of chondrocytes were not administered, compared to the joint (Normal) in which osteoarthritis was not induced, after the induction of osteoarthritis, but it was confirmed that the cartilage tissue lost due to osteoarthritis was supplemented in the joints (LYO and PCA) to which the culture medium-derived components of chondrocytes were administered after the induction of osteoarthritis.

Based on the results of tissue staining, the modified Mankin score was evaluated in order to explore the progress level of osteoarthritis (FIG. 3b). The Mankin score is used as an index to investigate the level of damage to articular cartilage by assessing four parameters including: cartilage structure, cellularity, proteoglycan depletion, and tide mark integrity.

FIG. 3b is a graph showing the results of comparing the modified Mankin score assessed for four types of joints (Normal, PBS, LYO, and PCA).

As shown in FIG. 3B, it was confirmed that the modified Mankin score showed a high level in the joint (PBS) to which the the culture medium-derived components of chondrocytes were not administered after the induction of osteoarthritis than in the joint (Normal) in which osteoarthritis was not induced, but the Mankin score showed a relatively low level in the joints (LYO and PCA) to which the culture medium-derived components of chondrocytes were administered after the induction of osteoarthritis. In addition, among the joints administered with the culture medium-derived components of chondrocytes, it was confirmed that the Mankin score showed a significantly low level in the joint (PCA) to which the PCA-dried components were administered than in the joint (LYO) to which the freeze-dried components were administered.

### Example 2-2-3: Assessment of Expression Levels of Osteoarthritis-Related Proteins

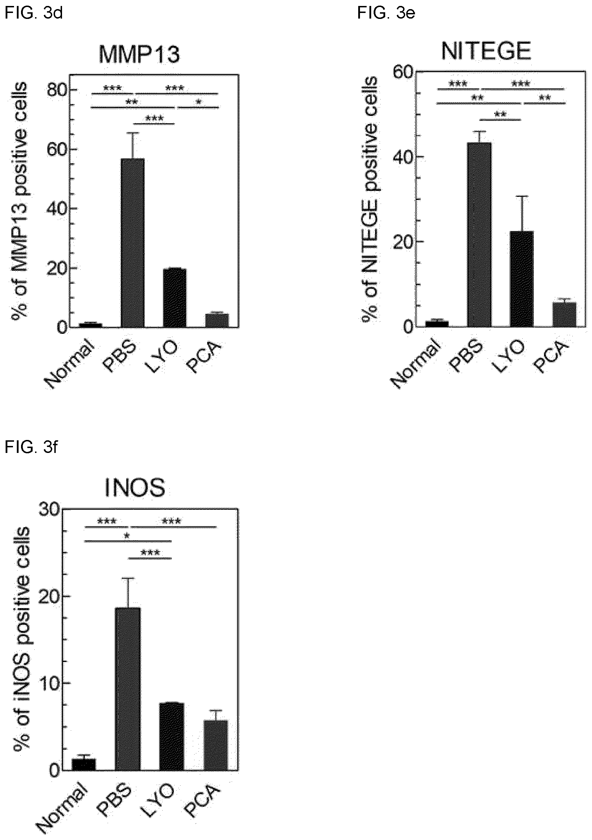

Immunofluorescence staining was performed for each joint region extracted in Example 2-2-1 (FIGS. 3c, 3d, 3e, and 3f) to compare the expression levels of osteoarthritis-related proteins (MMP13, NITEGE, and iNOS).

FIG. 3c is a fluorescence microscope showing the results of performing immunofluorescence staining assay to confirm the expression levels of osteoarthritis-related proteins (MMP13, NITEGE, and iNOS) in four types of joints (Normal, PBS, LYO, and PCA), FIG. 3d is a graph showing the results of measuring the ratio of cells, in which MMP13 was expressed, in the fluorescence micrograph, FIG. 3e is a graph showing the results of measuring the ratio of cells, in which NITEGE was expressed, in the fluorescence micrograph, and FIG. 3f is a graph showing the results of measuring the ratio of cells, in which iNOS was expressed, in the fluorescence micrograph.

As shown in FIGS. 3c, 3d, 3e, and 3f, it was confirmed that the expression levels of all three types of osteoarthritis-related proteins (MMP13, NITEGE, and iNOS) were increased in the joint (PBS) not administered with the culture medium-derived components of chondrocytes after the induction of osteoarthritis, compared to the joint (Normal) in which osteoarthritis was not induced, but the expression levels were decreased in the joints (LYO and PCA) to which the culture medium-derived components of chondrocytes were administered than in the joint (PBS) to which the culture medium-derived components of chondrocytes were not administered after the induction of osteoarthritis. In addition, it was confirmed that, among the joints to which the culture medium-derived components of chondrocytes were administered after the induction of osteoarthritis, the expression levels were relatively low in the joint (PCA) to which the PCA-dried components were administered than in the joint (LYO) to which the freeze-dried components were administered.

In summary of the results of *in vitro* and *in vivo* assays, the culture medium-derived components of chondrocytes show the effect of treating osteoarthritis caused by damage to the chondrocytes, and among the culture medium-derived components of chondrocytes, it can be found that the culture medium-derived components of chondrocytes obtained by the PCA drying method showed a relatively high level of osteoarthritis treatment effect than the culture medium-derived components of chondrocytes obtained by the freeze-drying method.

### Example 3: Optimization of PCA Drying Process

From the results of Example 2, since it was confirmed that the culture medium-derived components of chondrocytes obtained by the PCA drying method showed a relatively high level of osteoarthritis treatment effect compared to the culture medium-derived components of chondrocytes obtained by the freeze-drying method, it was attempted to establish PCA drying conditions capable of maximizing the production yield of the culture medium-derived components of chondrocytes when the PCA drying process is performed to obtain the culture medium-derived components of chondrocytes.

The present inventors made an attempt to establish the mixing ratio of each solvent constituting the co-solvent, the mixing ratio of the co-solvent and the culture medium of chondrocytes, the temperature condition, and the pressure condition, as the PCA drying conditions capable of maximizing the production yield of the culture medium-derived components of chondrocytes.

### Example 3-1: Mixing Ratio of Solvents Constituting Co-Solvent

When performing the PCA drying as in Example 1-3, since the co-solvent used is a mixed solvent of DMSO and acetone, it was investigated whether the yield of the culture medium-derived components of chondrocytes changes according to the mixing ratio of DMSO and acetone.

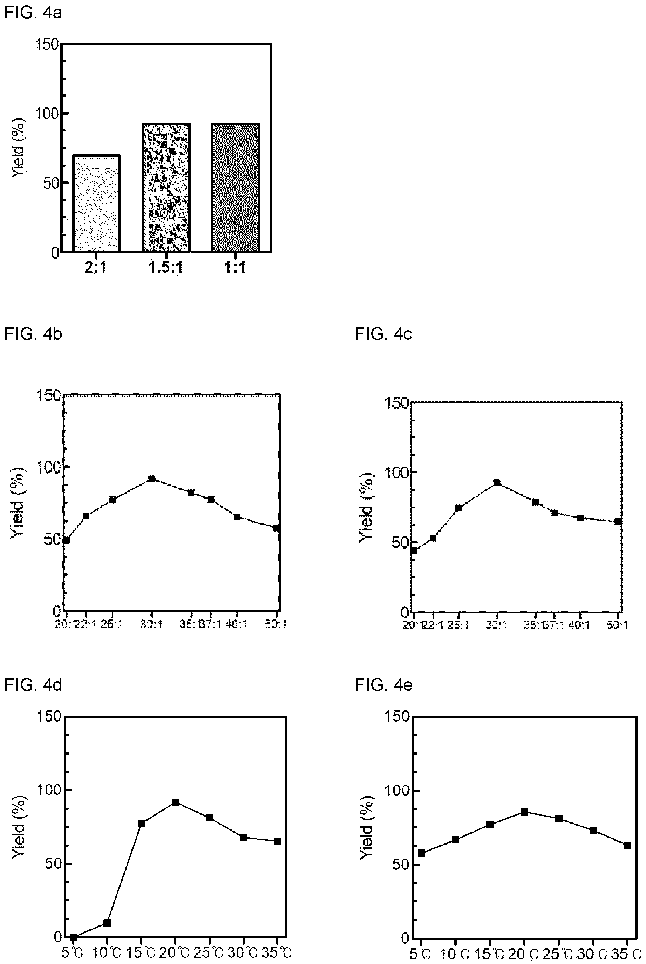

Roughly, respective co-solvents were prepared by mixing DMSO and acetone at a ratio of 2:1, 1.5:1, or 1:1 (v/v), and the respective co-solvents prepared above and the culture medium of chondrocytes were mixed at a ratio of 30:1 (v/v), and thereafter, the PCA drying process was performed under conditions of 20°C and 200 bar (FIG. 4a and Table 1).

FIG. 4a is a graph showing the results of comparing the change in yield of the culture medium-derived components of chondrocytes according to the mixing ratio of DMSO and acetone in performing the PCA drying process for the culture medium of chondrocytes.

**[Table 1]**

| DMSO:Acetone Ratio (v/v) | Co-solvent:Sample Ratio (v/v) | Tempera ture (°C) | Pressure (bar) | Yield (%) |
|---|---|---|---|---|
| 2:1 | 30:1 | 20 | 200 | 69.4 |
| 1.5:1 | | | | 92.5 |
| 1:1 | | | | 92.4 |

As shown in FIG. 4a and Table 1, it was confirmed that the yield of the culture medium-derived components of chondrocytes decreased when the DMSO content was in excess. In particular, it was confirmed that when the content of acetone was in excess, the culture medium-derived components of chondrocytes exhibited a precipitation-coagulation reaction and could be denatured.

Therefore, it was found that the preferred mixing ratio of DMSO and acetone was 2:1 to 1:1 (v/v)

### Example 3-2: Mixing Ratio of Co-Solvent and Culture Medium of Chondrocytes

When performing the PCA drying as in Example 1-3, since the mixture, in which the co-solvent and the culture medium of chondrocytes were mixed, was used, it was investigated whether the yield of the culture medium-derived components of chondrocytes changes according to the mixing ratio of the co-solvent and the culture medium of chondrocytes.

First, the co-solvent, in which the ratio of DMSO and acetone was 1.5:1 (v/v), and the culture medium of chondrocytes were mixed at a ratio of 20:1 to 50:1 (v/v), and then, the PCA drying process was performed under conditions of 20°C and 200 bar (FIG. 4b and Table 2).

FIG. 4b is a graph showing the results of comparing the change in yield of the culture medium-derived components of chondrocytes according to the mixing ratio of co-solvent, in which the mixing ratio of DMSO and acetone is 1.5:1 (v/v), and the culture medium of chondrocytes, in performing PCA drying process for the culture medium of chondrocytes.

**[Table 2]**

| DMSO:Acetone Ratio (v/v) | Co-solvent:Sample Ratio (v/v) | Tempera ture (°C) | Pressure (bar) | Yield (%) |
|---|---|---|---|---|
| 1.5:1 | 20:1 | 20 | 200 | 49.1 |
| | 22:1 | | | 65.9 |
| | 25:1 | | | 77.0 |
| | 30:1 | | | 91.8 |
| | 35:1 | | | 82.2 |
| | 37:1 | | | 77.4 |
| | 40:1 | | | 65.4 |
| | 50:1 | | | 57.6 |

As shown in FIG. 4b and Table 2, in the case of using the co-solvent, in which the ratio of DMSO and acetone is 1.5:1 (v/v), the yield of the culture medium-derived components of chondrocytes was decreased when the content of the co-solvent was excessively small or large. Additionally, it was confirmed that a yield of 70% or more was observed at the mixing ratio of 25:1 to 37:1 (v/v), and the maximum yield was observed at the mixing ratio of 30:1 (v/v).

Next, the co-solvent, in which the ratio of DMSO and acetone was 1:1 (v/v), and the culture medium of chondrocytes were mixed at a ratio of 20:1 to 50:1 (v/v), and then, the PCA drying process was performed under conditions of 20°C and 200 bar (FIG. 4c and Table 3).

FIG. 4c is a graph showing the results of comparing the change in yield of the culture medium-derived components of chondrocytes according to the mixing ratio of co-solvent, in which the mixing ratio of DMSO and acetone is 1:1 (v/v), and the culture medium of chondrocytes, in performing PCA drying process for the culture medium of chondrocytes.

**[Table 3]**

| DMSO:Acetone Ratio (v/v) | Co-solvent:Sample Ratio (v/v) | Tempera ture (°C) | Pressure (bar) | Yield (%) |
|---|---|---|---|---|
| 1:1 | 20:1 | 20 | 200 | 43.9 |
| | 22:1 | | | 52.9 |
| | 25:1 | | | 74.6 |
| | 30:1 | | | 92.4 |
| | 35:1 | | | 79.0 |
| | 37:1 | | | 71.3 |
| | 40:1 | | | 67.5 |
| | 50:1 | | | 64.8 |

As shown in FIG. 4c and Table 3, in the case of using the co-solvent, in which the ratio of DMSO and acetone is 1:1 (v/v), the yield of the culture medium-derived components of chondrocytes was decreased when the content of the co-solvent was excessively small or large. Additionally, it was confirmed that a yield of 70% or more was observed at the mixing ratio of 25:1 to 37:1 (v/v), and the maximum yield was observed at the mixing ratio of 30:1 (v/v).

In summary of the above results, in the range of the mixing ratio of DMSO and acetone established in Example 3-1, it was found that the preferred mixing ratio of the co-solvent and the culture medium of chondrocytes was 25:1 to 37:1 (v/v), and most preferred at 30:1 (v/v).

### Example 3-3: Temperature Conditions

When performing the PCA drying as in Example 1-3, it was investigated whether the yield of the culture medium-derived components of chondrocytes changes according to the temperature conditions.

First, the co-solvent, in which the ratio of DMSO and acetone was 1.5:1 (v/v), and the culture medium of chondrocytes were mixed at a ratio of 30:1 (v/v), and then, the PCA drying process was performed under conditions of 35°C and 200 bar (FIG. 4d and Table 4).

FIG. 4d is a graph showing the results of comparing the change in yield of culture medium-derived components of chondrocytes according to the temperature conditions when using a co-solvent, in which the mixing ratio of DMSO and acetone is 1.5:1 (v/v), in performing the PCA drying process for the culture medium of chondrocytes.

**[Table 4]**

| DMSO:Acetone Ratio (v/v) | Co-solvent:Sample Ratio (v/v) | Tempera ture (°C) | Pressure (bar) | Yield (%) |
|---|---|---|---|---|
| 1.5:1 | 30:1 | 5 | 200 | 0 (N/A) |
| | | 10 | | 9.8 |
| | | 15 | | 77.4 |
| | | 20 | | 91.8 |
| | | 25 | | 81.2 |
| | | 30 | | 67.9 |
| | | 35 | | 65.3 |

As shown in FIG. 4d and Table 4, in the case of using the co-solvent, in which the ratio of DMSO and acetone is 1.5:1 (v/v), it was confirmed that the yield of the culture medium-derived components of chondrocytes was decreased when the temperature was too high or too low. Additionally, it was confirmed that a yield of late 60% was observed at 15°C to 30°C, and the maximum yield was observed at 20°C.

Next, the co-solvent, in which the ratio of DMSO and acetone was 1:1 (v/v), and the culture medium of chondrocytes were mixed at a ratio of 28:1 (v/v), and then, the PCA drying process was performed under conditions of 5°C to 35°C and 150 bar (FIG. 4e and Table 5). In particular, the process conditions other than temperature, such as the ratio of DMSO and acetone, the mixing ratio of co-solvent and culture medium of chondrocytes, pressure, *etc.,* were set differently from those in FIG. 4d and Table 4, and accordingly, it was attempted to confirm whether similar tendency was observed under the temperature conditions while changing other process conditions.,

FIG. 4e is a graph showing the results of comparing the change in yield of culture medium-derived components of chondrocytes according to the temperature conditions when using a co-solvent, in which the mixing ratio of DMSO and acetone is 1:1 (v/v), in performing the PCA drying process for the culture medium of chondrocytes.

**[Table 5]**

| DMSO:Acetone Ratio (v/v) | Co-solvent:Sample Ratio (v/v) | Tempera ture (°C) | Pressure (bar) | Yield (%) |
|---|---|---|---|---|
| 1:1 | 28:1 | 5 | 150 | 57.7 |
| | | 10 | | 66.7 |
| | | 15 | | 77 |
| | | 20 | | 85.5 |
| | | 25 | | 81 |
| | | 30 | | 73 |
| | | 35 | | 63 |

As shown in FIG. 4e and Table 5, in the case of using the co-solvent, in which the ratio of DMSO and acetone is 1:1 (v/v), it was confirmed that the yield of the culture medium-derived components of chondrocytes was decreased when the temperature was too high or too low. Additionally, it was confirmed that a yield of 70% or more was observed at 15°C to 30°C, and the maximum yield was observed at 20°C.

In summary of the above results, in the range of the mixing ratio of DMSO and acetone established in Example 3-1, it was found that the preferred temperature condition was 15°C to 30°C, and most preferred at 20°C.

### Example 3-4: Pressure Conditions

When performing the PCA drying as in Example 1-3, it was investigated whether the yield of the culture medium-derived components of chondrocytes changes according to the pressure conditions.

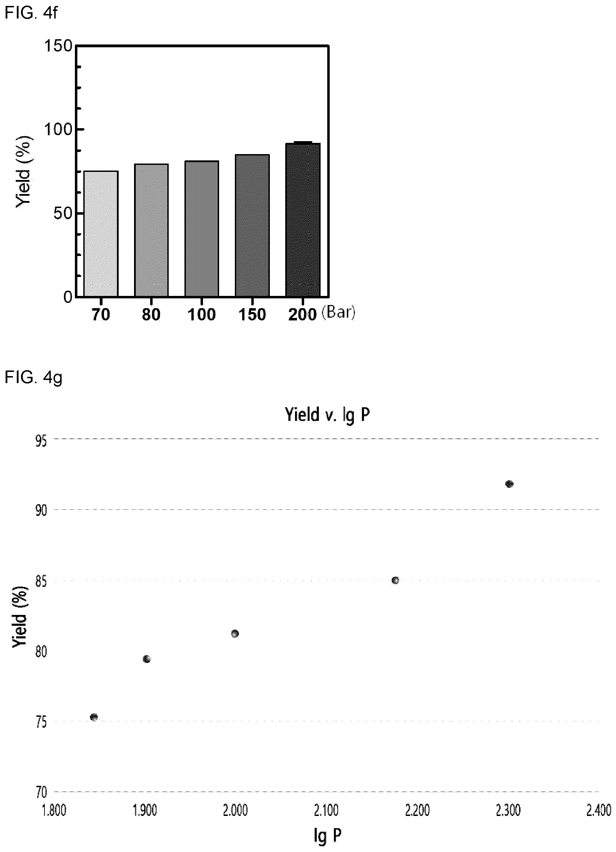

Roughly, the co-solvent, in which the ratio of DMSO and acetone was 1.5:1 (v/v), and the culture medium of chondrocytes were mixed at a ratio of 30:1 (v/v), and then, the PCA drying process was performed under conditions of 20°C and 70 bar to 200 bar (FIGS. 4f and 4g, and Table 4).

FIG. 4f is a graph showing the results of comparing the change in yield of culture medium-derived components of chondrocytes according to the pressure conditions when using a co-solvent, in which the mixing ratio of DMSO and acetone is 1.5:1 (v/v), in performing the PCA drying process for the culture medium of chondrocytes. FIG. 4g is a graph showing the above results in log scale.

**[Table 6]**

| DMSO:Acetone Ratio (v/v) | Co-solvent:Sample Ratio (v/v) | Tempera ture (°C) | Pressure (bar) | Yield (%) |
|---|---|---|---|---|
| 1.5:1 | 30:1 | 20 | 70 | 75.3 |
| | | | 80 | 79.4 |
| | | | 100 | 81.2 |
| | | | 150 | 85.0 |
| | | | 200 | 91.8 |

As shown in FIGS. 4f and 4g and Table 6, in the case of using the co-solvent, in which the ratio of DMSO and acetone is 1.5:1 (v/v), it was confirmed that the yield of the culture medium-derived components of chondrocytes was increased as the pressure increased, showing the maximum yield at 200 bar.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A method for preparing a culture medium-derived component or a crystallized culture medium exhibiting a high level of biological stability or activity, comprising:
(a) adding a co-solvent containing DMSO and acetone to a culture medium to obtain a mixture;
(b) adding a compressed anti-solvent to the mixture obtained above; and
(c) precipitating the culture medium-derived components in a pressure vessel.

2. The method of claim 1, wherein the co-solvent comprises DMSO and acetone in a ratio of 2:1 to 1:1.

3. The method of claim 1, wherein the co-solvent comprises DMSO and acetone in a ratio of 1.5:1 to 1:1.

4. The method of claim 1, wherein the co-solvent further comprises a solvent of an alcohol component.

5. The method of claim 1, wherein the mixture comprises the co-solvent and the culture medium in a ratio of 25:1 to 37:1.

6. The method of claim 1, wherein the mixture comprises the co-solvent and the culture medium in a ratio of 30:1 to 35:1.

7. The method of claim 1, wherein the temperature of the pressure vessel is 15°C to 30°C.

8. The method of claim 1, wherein the temperature of the pressure vessel is 20°C to 25°C.

9. The method of claim 1, wherein the pressure of the pressure vessel is 70 bar to 200 bar.

10. The method of claim 1, wherein the pressure of the pressure vessel is 200 bar.

11. The method of claim 1, wherein the compressed anti-solvent comprises at least one selected from the group consisting of carbon dioxide, dimethyl ether, and N₂O.

12. The method of claim 1, wherein the compressed anti-solvent comprises carbon dioxide.

13. The method of claim 1, wherein the culture medium is obtained by culturing chondrocytes in a medium and collecting the medium.

14. The method of claim 1, wherein the culture medium contains growth factors, extracellular matrix, extracellular vesicles, chemokines, and cytokines.

15. The method of claim 1, wherein the precipitation comprises crystallization.

16. A pharmaceutical composition for preventing or treating osteoarthritis, comprising the culture medium-derived component or the crystallized culture medium prepared by way of the method of any one of claims 1 to 15, as an active ingredient.

17. The pharmaceutical composition of claim 16, wherein the composition further comprises a pharmaceutically acceptable carrier, excipient, or diluent.

18. A method for restoring chondrocytes, comprising treating chondrocytes with the culture medium-derived component or the crystallized culture medium prepared by way of the method of any one of claims 1 to 15.

19. The method of claim 18, wherein the method alleviates the inflammatory environment of chondrocytes.

20. The method of claim 18, wherein the method suppresses the expression of catabolism-related genes of chondrocytes.

21. A method for treating a subject having osteoarthritis, comprising administering the culture medium-derived component or the crystallized culture medium prepared by way of the method of any one of claims 1 to 15 to a subject other than human having osteoarthritis.

22. The method of claim 21, wherein the administration comprises injecting the culture medium-derived component into the site where osteoarthritis has occurred in the subject.
